# EUROPEAN PATENT SPECIFICATION

(11) **EP 1 389 967 B1**
(45) Date of publication and mention of the grant of the patent: **31.01.2007**
(21) Application number: 01959565.1
(22) Date of filing: 07.08.2001
(51) Int. Cl.: A61F 9/01

(54) **APPARATUS FOR TREATMENT OF PRESBYOPIA AND OTHER EYE DISORDERS USING FIBER-COUPLED-LASERS**
APPARAT ZUR BEHANDLUNG VON PRESBYOPIE UND ANDERER AUGENERKRANKUNGEN MIT FASER-GEKOPPELTEN LASERN
APPAREIL DE TRAITEMENT DE LA PRESBYTIE ET D'AUTRES AFFECTIONS DE L'OEIL, METTANT EN OEUVRE DES LASERS COUPLES PAR FIBRES

(30) Priority: 06.11.2000 US 706382
(43) Date of publication of application: 25.02.2004
(73) Proprietor: SURGILIGHT,INC., Orlando, Florida 32826 (US)
(72) Inventor: Lin, J.-T., Oviedo, FL 32765 (US)
(74) Representative: Whitaker, Iain Mark
(86) International application number: PCT/US2001/024618
(87) International publication number: WO 2002/036029

(56) References cited:
- EP-A- 0 689 811
- WO-A-98/41177
- US-A- 4 907 586
- US-A- 5 354 331
- US-A- 5 423 801
- US-A- 6 090 100
- US-A- 6 099 522

## Description

### BACKGROUND OF THE INVENTION

### 1. Field of the Invention

The present invention relates to apparatus for the treatment of presbyopia, glaucoma and cataract, using fiber-coupled and scanning lasers and non-laser devices to ablate the sclera tissue.

### 2. Prior Art

Corneal reshaping including a procedure called photorefractive keratectomy (PRK) and a new procedure called laser assisted in situ keratomileusis, or laser intrastroma keratomileusis (LASIK) have been performed by lasers in the ultraviolet (UV) wavelength of (193 - 213) nm. The commercial UV refractive lasers include ArF excimer laser (at 193 nm) and other non-excimer, solid-state lasers such as those proposed by the present inventor in 1992 (US pat. no. 5,144,630) and in 1996 (US pat. No. 5,520,679). The above-described prior arts using lasers to reshape the corneal surface curvature, however, are limited to the corrections of myopia, hyperopia and astigmatism.

Refractive surgery using a scanning device and lasers in the mid-infrared (mid-IR) wavelength was first proposed by the present inventor in US Pat. No. 5,144,630 and 5,520,679 and later proposed by Telfair et. al., in US Pat. No. 5,782,822, where the generation of mid-IR wavelength of (2.5-3.2) microns were disclosed by various methods including: the Er:YAG laser (at 2.94 microns), the Raman-shifted solid state lasers (at 2.7-3.2 microns) and the optical parametric oscillation (OPO) lasers (at 2.7-3.2 microns).^{a}

Corneal reshaping may also be performed by laser thermal coagulation currently conducted by a Ho:YAG laser (at about 2 microns in wavelength) proposed by Sand in US Pat. 5,484,432. This method, however, was limited to low-diopter hyperopic corrections. Strictly speaking this prior art did not correction the true "presbyopia" and only performed the mono-vision for hyperopic patients. A thermal laser is required and the laser treated area was within the optical zone diameters of about 7 mm.

Ruiz in US pat. No. 5,533,997 proposed the use of laser ablation of cornea surface to correct presbyopic patients. This prior art, however, must generate multifocal (or bifocal) surface on the central portion of the cornea in order to achieve the desired presbyopia correction. Corneal curvature change by laser ablation in this prior art, however, did not actually resolve the intrinsic problems of presbyopic patient caused by age where the cornea lens loss its accommodation as a result of loss of elasticity due to age.

All the above-described prior arts are using methods to change the cornea surface curvature either by tissue ablation (such as in UV laser) or by thermal shrinkage (such as in Ho:YAG laser) and all are using lasers onto the central potion of the cornea.

The alternative method for presbyopia correction, therefore, is to increase the accommodation of the presbyopic patients by change the intrinsic properties of the sclera and cililary tissue to increase the lens accommodation without changing the cornea curvature. This method of sclera ablation is fundamentally different from all the prior arts including that of Ruiz, in which reshaping cornea curvature into multifocal shape was required for presbyopia correction.

To treat presbyopic patients, or the reversal of presbyopia, using the concept of expanding the sclera by mechanical devices has been proposed by Schachar in U.S. patents 5,489,299, 5,722,952, 5,465,737 and 5,354,331. These mechanical approaches have the drawbacks of complexity and are time consuming, costly and have potential side effects. To treat presbyopia, the Schachar patents Nos. 5,529,076 and 5,722,952 propose the use of heat or radiation on the corneal epithelium to arrest the growth of the crystalline lens and also propose the use of lasers to ablate portions of the thickness of the sclera. However, these prior arts do not present any details or practical methods or laser parameters for the presbyopic corrections. No clinical studies have been practiced to show the effectiveness of the proposed concepts. The concepts proposed in the Schachar patents, 5,354,331 and 5,489,299, regarding lasers suitable for ablating the sciera tissues were incorrect because he did not identify which lasers are "cold lasers". Many of his proposed lasers are thermal lasers which will cause thermal burning of the cornea, rather than tissue ablation. Furthermore, the clinical issues, such as locations, patterns and depth of the sclera tissue removal were not indicated in these prior patents. In addition, it is essential to use a scanning or fiber-coupled laser to achieve the desired ablation pattern and to control the ablation depth on the sclera tissue. Schachar's methods also require the weakening of the sclera and increase its diameter by expansion, whereas the proposed concept of the present invention provides new mechanisms for accommodation.

The "presbyopia" correction proposed by Ruitz (US Pat. No. 5,533,997) using an excimer (ArF) laser also required the corneal surface to be reshaped to form "multifocal" effort for a presbyopia patents to see near and far. However, Ruitz's "presbyopia" correction is fundamentally different from that of the present patent which does not change the corneal curvature. The presbyopia correction proposed in the present patent is to increase patient's accommodation rather than reshaping the cornea into "multifocal" surface.

The technique used in the prior art of Bille (Pat. No. 4,907,586) required a quasi- continuous laser having pulse duration less than 10 picoseconds and focused spot less than 10 micron diameter and the laser is confined to the interior of a selected tissue to correct myopia, hyperopia or astigmatism. Bille also proposed the laser to focused into the lens of an eye to prevent presbyopia. This prior art system is very complicate and needs a precise control of the laser beam size and focusing position. Furthermore, clinical risk of cataract may occur when laser is applied into the lens area.

Another prior art proposed by Spencer Thornton (Chapter 4, "Surgery for hyperopia and presbyopia", edited by Neal Sher (Williams & Wilkins, MD, 1997) is to use a diamond knife to incise radial cuts around the limbus areas. It requires a deep (90%-98%) cut of the sclera tissue in order to obtain accommodation of the lens. This method, however, involves a lot of bleeding and is difficult to control the depth of the cut which requires extensive surgeon's skill. Another drawback for presbyopia correction provided by the above-described incision methods is the major post-operative regression of about (30%-80%). And this regression is minimum in the laser-ablation method proposed in the present invention. We note that there is intrinsic difference between the ablation-method proposed in this invention and the knife-incision. The sclera space produced by the incision method is not permanent and may be greatly reduced during the tissue healing and cause the regression. This major source of regression in incision method however will not occur in the laser or non-laser ablation-methods as proposed in this invention, where portion of the sclera tissue is permanently removed.

The important concept proposed in the present invention is to support the post-operative results which show minimum regression. We proposed that the laser ablated sclera tissue "gap" will be filled in by the sub-conjunctival tissue within few days after the surgery. This filled in sub-conjunctival tissue is much more flexible than the original sclera tissue. Therefore the filled-in gap in the sclera area will cause the underlaying ciliary body to have more space to move. This in turn will allow the ciliary body to contract or expand the zonular fiber which is connected to the lens, when the presbyopic patient is adjusting his lens curvature to see near and far. The above described sub-conjunctival tissue filling effects and the increase of "flexibility" of the sclera area are fundamentally different from the scleral "expansion"(or weakening) concept proposed by the prior arts of Schachar and proposed by the implant of a scleral band. In the present invention, the laser ablated sclera area is not weakening, it becomes more flexible instead.

One objective of the present invention is to provide an apparatus and method to obviate these drawbacks in the prior arts.

It is yet another objective of the present invention to use a scanning device and fiber-coupled system such that the degree of ciliary mussel accommodation can be controlled by the ablation patterns, location, size and shapes of the removed sclera tissue.

It is yet another objective of the present invention to define the non-thermal lasers for efficient tissue ablation.

It is yet another objective of the present invention to define the optimal laser parameters and the ablation patterns for best clinical outcome for presbyopia patients, where sclera ablation will increase the accommodation of the ciliary mussel by the increase of the flexibility in the laser-ablated areas.

It is yet another objective of the present invention to provide the appropriate ablation patterns which will cause effective ciliary body contraction and expansion on the zonuies and the lens.

It is yet another objective of the present invention to provide a new mechanism which supports the clinical results of presbyopia correction with minimum regression. One important concept proposed in the present invention is to support the post-operative results which show minimum regression when presbyopia is corrected by a laser ablation for the sclera tissue.

We proposed that the laser ablated sclera tissue "gap" will be filled in by the sub-conjunctival tissue within few days after the surgery. This filled in sub-conjunctival tissue is much more flexible than the original sclera tissue. Therefore the filled-in gap in the sclera area will cause the underlaying ciliary body becomes more flexible. This will allow the ciliary body to contract or expand the zonular fiber connected to the lens when the presbyopic patient is adjusting his lens curvature to see near and far.

The concept presented in the present patent is to remove portion of the sclera tissue which is filled in by sub-conjunctiva tissue to increase the flexibility of the scleral area and in turn causes the zonular fiber to increase the lens accommodation. Therefore the laser ablation effects on the scleral tissue may also be conducted by any non-laser methods which removes the scleral tissue at a width about (0.2-2.0) mm and length of (2.0-5.0) mm, as far as this area can be filled in by the sub-conjunctival tissue. These non-laser methods shall include, but not limited to, physical blades or knife, electromagnetic wave such as radio frequency wave, electrode device, bipolar device and plasma assisted electrosurgical device.
Another important concept proposed in the present invention is to support the post-operative results which show minimum regression. We proposed that the laser ablated sclera tissue "gap" will be filled in by the sub-conjunctival tissue within few days after the surgery. This filled in sub-conjunctival tissue is much more flexible than the original sclera tissue. Therefore the filled-in gap in the sclera area will cause the underneath ciliary body to contract or expand the zonular fiber connected to the lens when the presbyopic patient is adjusting his lens curvature to see near and far.

The present invention described in great detail for the treatment of presbyopia may be extended to other eye disorders including glaucoma and cataracts. For the case of glaucoma, the laser and non-laser devices may be used to remove sclera tissue in the area where Schlemm's channel is located followed by a removal of a small portion of the iris underlying this area. For the treatment of cataracts, one needs to perform a scleral partial thickness incision in order for a ultrasound device to go through to the lens. Using the proposed laser and non-laser ablation-method provides many advantages over the currently used diamond blades.

The invention having now been fully described, it should be understood that it may be embodied in other specific forms or variations without departing from the spirit or essential characteristics of the present invention. Accordingly, the embodiments described herein are to be considered to be illustrative and not restrictive.

Further background art is provided in WO 98/41177 A (IRVISION, INC.) and US-A-4,907,586 (Bille et al.). WO 98/41177 A discloses a laser parametric generator for surgical applications which utilizes short-pulse, mid-infrared radiation. The mid-infrared radiation may be produced by a pump laser source, such as a neodymium-doped laser, which is parametrically downconverted in a suitable nonlinear crystal to the desired mid-infrared range. The short pulses reduce unwanted thermal effects and changes in adjacent tissue to potentially submicron levels. A fiber, fiber bundle or another waveguide means utilized to separate the pump laser from the optical parametric oscillation (OPO) cavity is also disclosed.

US-A-4,907,586 discloses a method for modifying tissue with a quasi-continuous laser beam to change the optical properties of the eye, comprising controllably setting the volumetric power density of the beam and selecting a desired wavelength for the beam. Tissue modification is accomplished by focusing the beam at a preselected start point in the tissue and moving the beam's focal point in a predetermined manner relative to the start point throughout a specified volume of the tissue or along a specified path in the tissue. Depending on the selected volumetric power density, the tissue on which the focal point is incident can be modified either by photoablation or by a change in the tissue's visco-elastic properties.

### SUMMARY OF THE INVENTION

The invention is a surgical apparatus as defined in Claim 1 of the appended claims.

The preferred embodiments of the basic surgical lasers of the present invention shall include: (a) infrared (IR) lasers having wavelengths range of about (1.4 - 3.2) microns including but not limited to solid state lasers of Er:glass, Ho:YAG, Er:YAG, Er:YSGG, infrared gas lasers, solid-state lasers converted by optical parametric oscillation (OPO); (b) ultraviolet (UV) lasers having wavelength range of about (190 - 355) nm, such as ArF (at 193 nm) and XeCl (at 308 nm) excimer lasers, nitrogen laser (at 337 nm) and solid-state lasers using frequency conversions; (c) semiconductor diode lasers at about 980 nm, (1.3-1.55) microns, and (1.8-2.1) microns; (d) flash-lamp-pumped and diode-pumped solid state lasers having wavelength range of about (190-355) nm and (2.7-3.2) microns such as Er:YSGG, Er:YAG, Nd:YAG, Er:glass andTi:saphire laser and their harmonic generation; (e) short pulse infrared lasers with pulse duration of between about 1.0 femtosecond and 100 picoseconds.

It is yet another preferred embodiment is to couple the basic lasers by a fiber and deliver the laser beam to the treated area of the eye by a hand held piece which is further connected to a fiber-tip at various shapes.

It is yet another preferred embodiment to focus the laser beams into a desired spot size on the treated area of the eye. Various ablation patterns may be generated manually via the fiber-connected hand piece including multiple rings of spots and radial line incisions outside the limbus.

It is yet another preferred embodiment to focus the laser beams into a means of scanning device such that various ablation patterns may be generated by controlling the scanning device. The scanning devices shall include the use of a motorized reflection mirror, refractive optics device or manually controlled translation device.

It is yet another preferred embodiment is to remove, by any methods either laser or non-laser, portion of the sclera tissue which is filled in by sub-conjunctiva tissue to increase the flexibility of the scleral area and in turn causes the zonular fiber to increase the lens accommodation. The preferred embodiment for non-laser methods shall include, but not limited to, physical blades or knife, electromagnetic wave such as radio frequency wave, electrode device, bipolar device and plasma assisted electrode device.

It is yet another preferred embodiment to open the conjunctiva layer prior to the laser ablation of the under-layer of the sclera tissue for a better control of the ablation depth and for safety reasons. It is yet another preferred embodiment is that the conjunctiva layer may be lifted to generate the "gap" for fiber tip to insert into the gap and ablate the desired patterns underneath and to avoid or minimize bleeding or infection.

Further preferred embodiments of the present invention will become apparent from the description of the invention which follows.

### BRIEF DESCRIPTION OF THE DRAWINGS

FIG. 1 is a block diagram of the integrated laser system consisting of a laser, a coupling fiber delivery unit and a hand-piece connected to a fiber-tip to control the beam spot on the treated area.
FIG. 2 shows various shapes of the fiber tips: (A) flat tip, (B) spherical tip for focused contact use, (C) conical tip, (D) 90-degree angle tip, and (E) focused slit-spot.
FIG. 3 shows various ablation patterns generated by the ablating laser outside the limbus.

### DETAILED DESCRIPTION OF THE INVENTION AND THE

### PREFERRED EMBODIMENTS

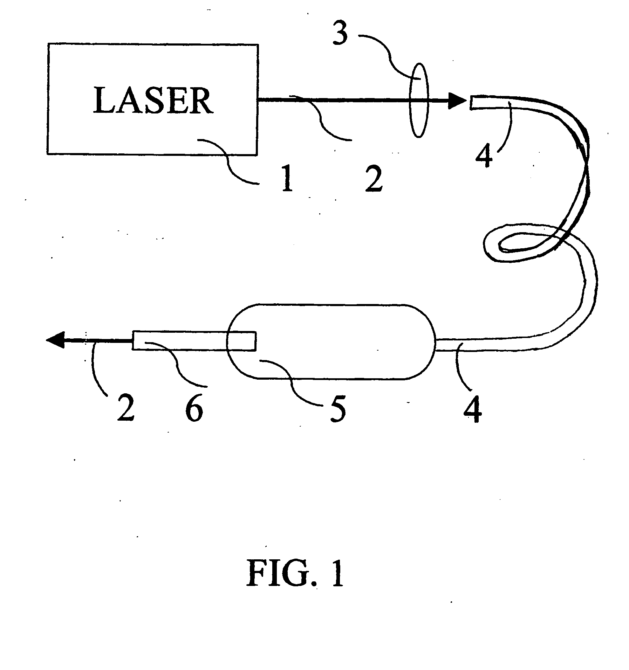

Referring to Fig. 1, a surgical laser system in accordance with the present invention comprises a basic laser 1 having wavelength 2 coupled by a focusing lens 3 to a fiber 4 which is connected to a hand-piece 5 and a fiber tip 6. The focusing lens 3, fiber 4 and fiber tip 6 are highly transparent to the wavelength 2 of the basic laser.

Still referring to Fig. 1, according to the present invention, the preferred embodiments of the basic surgical lasers for presbyopia correction procedures shall include: (a) infrared lasers having wavelengths range of about (1.4 - 3.2) microns including but not limited to solid state lasers of Er:glass, Ho:YAG, Er:YAG, Er:YSGG, infrared gas lasers, solid-state lasers converted by optical parametric oscillation (OPO); (b) ultraviolet (UV) lasers having wavelength range of about (190 - 360) nm, such as ArF (at 193 nm) and XeCl (at 308 nm) excimer lasers, nitrogen laser (at 337 nm) and solid-state lasers using harmonic generation form solid-state lasers of Nd:YAG, Nd:YLF and Alexandrite lasers frequency conversions; (c) semiconductor diode lasers at about 980 nm, (1.3-1.55) microns, and (1.8-2.1) microns; (d) diode-pumped solid state lasers having wavelength range of about (190-355) nm and (2.7-3.2) microns such as diode-pumped Er.YSGG, Er:YAG, Nd:YAG and Er:glass; (e) diode lasers having wavelength at about 980 nm, 1.5 microns, and 1.9 microns; (f) short pulsed laser at about (0.5 - 1.2) microns with pulse width less than 100 picoseconds.

According to one aspect of the present invention, the preferable scanning laser energy per pulse on scleral surface is about (1-40) mJ in IR lasers and about (0.1 - 5.0) mJ in UV lasers. Focused spot size of about (0.1-1.0) mm in diameter on the scleral plane is achieved by the focusing lens 3 which consists of at least one spherical lens. The other preferred laser parameter of this invention is the laser repetition rate range of about (5-100) Hz which will provide reasonable surgical speed and minimum thermal effects. The focused beam may be scanned over the scleral surface to ablate various patterns.

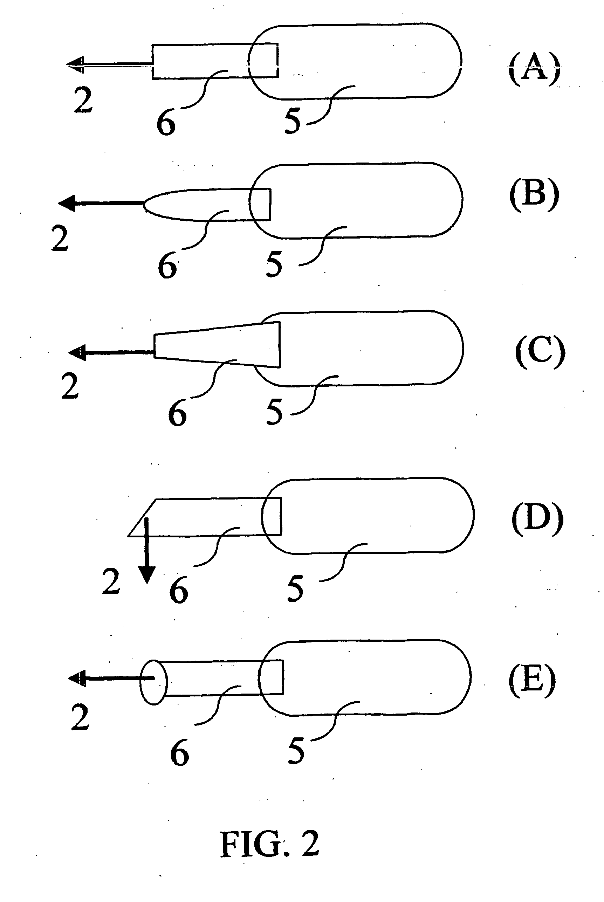

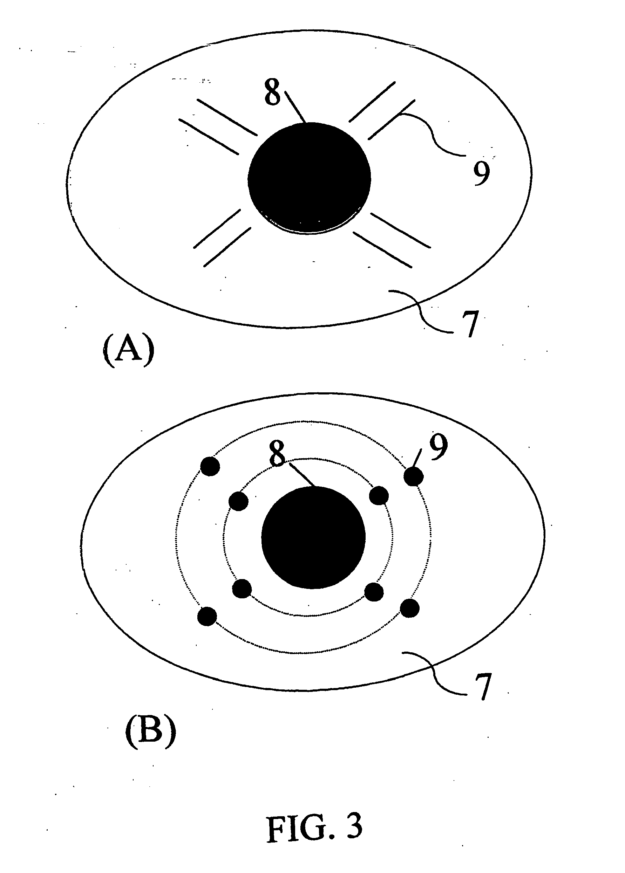

Referring to Fig. 2(A), the laser output from the fiber end having wavelength 2 is connected to the hand-piece 5 and a flat fiber tip 6 such that the output laser beam from the end of the fiber tip is a round-beam with a predetermined spot size of about (0.1- 1.0) mm. Fig. 2(B) shows similar structure to Fig. 2(A), except the output round-spot beam is re-focused by the spherical shape of the tip. Fig. 3 (C) shows the output beam 2 is guided by a conical shape tip such that the beam size at the end of the tip is reduced. Fig. 2(D) shows that the output beam is reflected by 90-degree by a coated fiber tip. Finally Fig. 2(E) shows an output beam spot is a siit-shape having a size of about (0.1-1.0) x (1.5-5.0) mm formed by a cylinder lens attached to the end of the fiber tip.

Fig. 3 shows an eye 7 of a presbyopic patient with ablation patterns 9 generated on the scleral area about (0.1-1.0) mm posterior to the corneal limbus 8. The preferred patterns of this invention include a ring-spot having at least one ring with at least 3 spots in each ring, and a radial-pattern having at least 3 radials. The preferred area of the ablation is defined within two circles having diameters about 10 mm and 14 mm posterior to the limbus along the radial direction of the cornea. We should note that a radial ablation pattern on the scleral surface may be generated either by an automatic scanning device or by manually scan the fiber tip by a surgeon who hold the hand piece. For the situation of the slit fiber-tip, the surgeon may easily generate the radial patterns without moving the tip.

The ablation depth of the sclera ciliary tissue is about (400-700) microns with each of the radial length of about (2.5 - 5.0) mm adjustable according to the optimal clinical outcomes including minimum regression and maximum accommodation for the presbyopic patients. The preferred radial ablation shall start at a distance about (4.0 - 5.5) mm from the corneal center and extended about (2.0-5.0) mm outside the limbus. The preferred embodiments of the radial patterns on the sclera area include at least 3 radial lines, curved lines, ring-dots or any non-specific shapes in a symmetric geometry as shown in Fig. 3.

Still referring to Fig. 3, the preferred embodiments to generate the radial patterns on the sclera area include the following examples. Examples (a): Scan the round laser spot of about (0.1- 1.0) mm in diameter produced from the fiber tips in the radial directions to generate each of the radial lines. Generation of the radial patterns may be done either manually moving the fiber tip along the sclera radial direction or by an automatically a scanner or translator. Example (b): Use a focused slit-beam to generate the radial lines. In case (b), a scanning device is not needed and each of the radial lines may be generated by the slit beam directly. Any other non-specific patterns including curved lines, z-shape, t-shape lines around the area outside the limbus should be within the scope of this patents.

It is yet another preferred embodiment to focus the laser beams into a means of scanning device such that various ablation patterns may be generated by controlling the scanning device. The scanning device shall include the use of a motorized reflection mirror, refractive optics device or manually controlled translation device.

One preferred embodiment is to coagulate the conjunctiva layer and then cut (by a knife) a half-circle over the conjunctiva surrounding the limbus with a diameter about 10 mm which is then pushed aside in order for the ablating laser to cut the sclera layer underneath. It is also possible to use the ablating laser to cut the conjunctiva layer which however may take a longer time than cutting by a knife.

Another preferred embodiment is not to open the conjunctiva layer, but to insert the fiber tip through the conjunctiva layer and ablate the sclera tissue underneath such that the procedure is done non-invasively. To do this procedure, the conjunctiva layer may be lifted to generate the "gap" for fiber tip to insert into the gap and ablate the desired patterns underneath. Additional advantages of this invasive method is to avoid or minimize bleeding or infection. We note that most of the bleeding is due to cutting of the conjunctiva tissue rather than the laser ablation of the sclera tissue.

It is yet another preferred embodiment is to remove, by non-laser methods, portion of the sclera tissue which is filled in by sub-conjunctiva tissue to increase the flexibility of the scleral area and in turn causes the zonular fiber to increase the lens accommodation. The preferred embodiment for these non-laser methods shall include, but not limited to, physical blades or knife, electromagnetic wave such as radio frequency wave, electrode device, bipolar device and plasma assisted electrode device. The electromagnetic wave generator is commercially available. However, the parameters of the device such as its frequency, pulse duration and repetition rate and the size of the electrode tip shall be selected for efficient cutting (or ablation) with minimum thermal damage to the tissue to be removed.

It is yet another preferred embodiment is to remove, by laser of non-laser methods the sclera tissue for the treatment of other eye disorders including prebyopia; glaucoma (by reducing the intraocular pressure) and cataracts (to prepare the opening).

While the invention has been shown and described with reference to the preferred embodiments thereof, it will be understood by those skilled in the art that the foregoing and other changes and variations in form and detail may be made therein without departing from the scope of the invention as defined in the appended claims. Accordingly, threshold and apparatus, the ophthalmic applications herein disclosed are to be considered merely as illustrative and the invention is to be limited only as set forth in the claims.

## Claims

1. A surgical apparatus for treating a presbyopic patient by removing a portion of the sclera tissue of an eye comprising:
(a) a laser for emitting a laser beam (1) having a predetermined wavelength (2); and
(b) a beam spot controller mechanism (3), said beam spot controller being adapted to reduce and focus said laser beam (1) to a fiber delivery unit (4,5,6);
the surgical apparatus **characterised by**:
(c) a control means adapted to control said fiber delivery unit (4,5,6) to deliver said laser beam (1) in a predetermined pattern (9) onto a plurality of positions on the eye and to ablate the sclera tissue outside the limbus area to a depth of between about 400 µm and 700 µm,
whereby the apparatus is adapted to correct a presbyopic patient's vision to see near by increasing the accommodation of the eye through the increase in flexibility of the ablated area being filled by sub-conjunctival tissue.

2. A surgical apparatus as claimed in claim 1, wherein said laser beam (1) is emitted by an ultraviolet laser having a wavelength range of about (0.15 - 0.36) microns and a pulse duration less than about 200 nanoseconds or an infrared laser having a wavelength range of about (1.4 - 3.2) microns or a solid-state diode laser having a wavelength range of about (0.95 - 2.1) microns or an ArF excimer laser having a wavelength of 193 nm or a XeCl excimer laser having a wavelength of 308 nm.

3. A surgical apparatus as claimed in claim 2, wherein the infrared laser is an optically pumped Erbium:YAG laser having a wavelength of about 2.9 microns, or an optically pumped Er:YSGG laser having a wavelength of about 2.7 microns.

4. A surgical apparatus as claimed in claim 1, in which said beam spot controller consists of at least one focusing spherical lens (3) to couple said laser beam (1) to said fiber delivery unit (4,5,6).

5. A surgical apparatus as claimed in claim 1, wherein said fiber delivery unit (4,5,6) consists of an optical fiber (4) having a length of about (0.5 - 1.5) meter and core diameter of about (0.2 - 0.8) mm and a hand piece connected to the fiber tip.

6. A surgical apparatus as claimed in claim 5, wherein said fiber delivery unit (4,5,6) is substantially transparent to the wavelength of said laser beam (1).

7. A surgical apparatus as claimed in claim 5, wherein said fiber tip (6) is made of a similar material as that of the fiber (4) and is made in one of the following shapes to focus said laser beam (1) onto the treated sclera area of the eye (7): conical, spherical, 90-degree reflecting angle and flat end.

8. A surgical apparatus as claimed in claim 5, wherein said fiber tip (6) focuses said laser beam (1) onto the treated area of the eye (7) at a spot size of about 0.1 - 1.0) mm in diameter.

9. A surgical apparatus as claimed in claim 5, wherein said fiber tip (6) is made in a cylinder shape to focus said laser beam (1) onto the treated area of the eye (7) at a line shape having a dimension of about (0.1 - 0.4) mm in width and (0.5 - 4.0) mm in length.

10. A surgical apparatus as claimed in claim 5, wherein said fiber tip (6) is operated in a contact-mode or a non-contact mode to ablate the sclera tissue to a depth of about (300 - 800) microns.

11. A surgical apparatus as claimed in claim 1, wherein said fiber delivery unit (4,5,6) is controllable by the surgeon to perform said predetermined patterns (9) outside the limbus (8) of the cornea by manually moving said fiber tip (6) in the radial direction of the eye.

12. A surgical apparatus as claimed in claim 1, wherein said fiber delivery unit (4,5,6) is attached to a scanning device to perform said predetermined patterns (9) outside the limbus (8) of the cornea and scan said laser beam (1) along the radial direction of the eye.

13. A surgical apparatus as claimed in claim 1, wherein said predetermined patterns are outside the limbus (8) of the cornea and defined by the area between two circles having radius of about 5.0 mm and 9.0 mm, respectively.

14. A surgical apparatus as claimed in claim 1, wherein said predetermined pattern (9) includes radial lines, curved lines, ring-dot or non-specific patterns around the area outside the limbus (8).

15. A surgical apparatus as claimed in claim 1, wherein said ablation laser comprises an ArF or XeCl excimer laser, or an Er:YAG laser or an Er:YSGG, or a Nd:YAG laser, or a Nd:YLF laser.

16. A surgical apparatus as claimed in claim 1, wherein said control means includes a scanning device having motorized reflection mirror, or refractive optics, or translation device.

## Patentansprüche

1. Chirurgische Vorrichtung zur Behandlung eines presbyopischen Patienten durch Entfernen eines Teils des Skleragewebes eines Auges, umfassend:
(a) einen Laser zum Ausstrahlen eines Laserstrahls (1), der eine vorbestimmte Wellenlänge (2) hat; und
(b) einen Strahlenpunkt-Steuermechanismus (3), wobei die Strahlenpunktsteuerung angepasst ist zur Reduzierung und Fokussierung des Laserstrahls (1) auf eine Faserzuführungseinheit (4,5,6);
die chirurgische Vorrichtung charakterisiert durch:
(c) ein Steuermittel angepasst zur Steuerung der Faserzuführungseinheit (4,5,6) zur Zuführung des Laserstrahls (1) in einem vorbestimmten Muster (9) auf eine Pluralität von Positionen auf dem Auge und zum Abladieren des Skleragewebes außerhalb des Limbusbereichs auf eine Tiefe zwischen etwa 400 µm und 700 µm,
wobei die Vorrichtung angepasst ist zur Korrektur des Nahsehvermögens eines presbyopischen Patienten durch Erhöhung der Akkommodation des Auges durch die Erhöhung der Flexibilität des abladierten Bereichs, der durch subkonjunktivales Gewebe gefüllt ist.

2. Chirurgische Vorrichtung nach Anspruch 1, wobei der Laserstrahl (1) von einem Ultraviolettlaser mit einem Wellenlängenbereich von etwa (0,15 - 0,36) Mikrometer und einer Impulsdauer von weniger als etwa 200 Nanosekunden oder einem Infrarotlaser mit einem Wellenlängenbereich von etwa (1,4 - 3,2) Mikrometer oder einem Festkörperdiodenlaser mit einem Wellenlängenbereich von etwa (0,95 - 2,1) Mikrometer oder einem ArF-Excimerlaser mit einer Wellenlänge von 193 nm oder einem XeCl-Excimerlaser mit einer Wellenlänge von 308 nm ausgestrahlt wird.

3. Chirurgische Vorrichtung nach Anspruch 2, wobei der Infrarotlaser ein optisch gepumpter Erbium:YAG-Laser mit einer Wellenlänge von etwa 2,9 Mikrometer oder ein optisch gepumpter Er:YSGG-Laser mit einer Wellenlänge von etwa 2,7 Mikrometer ist.

4. Chirurgische Vorrichtung nach Anspruch 1, in der die Strahlenpunkt-Steuerung mindestens eine sphärische Fokussierlinse (3) umfasst, um den Laserstrahl (1) mit der Faserzuführungseinheit (4,5,6) zu koppeln.

5. Chirurgische Vorrichtung nach Anspruch 1, in der die Faserzuführungseinheit (4,5,6) eine optische Faser (4) mit einer Länge von etwa (0,5 - 1,5) Meter und einem Kerndurchmesser von etwa (0,2 - 0,8) mm und ein Handstück, das mit der Faserspitze verbunden ist, umfasst.

6. Chirurgische Vorrichtung nach Anspruch 5, in der die Faserzuführungseinheit (4,5,6) im Wesentlichen für die Wellenlänge des Laserstrahls (1) transparent ist.

7. Chirurgische Vorrichtung nach Anspruch 5, in der die Faserspitze (6) aus einem ähnlichen Material wie das der Faser (4) hergestellt ist und in einer der folgenden Formen hergestellt ist, um den Laserstrahl (1) auf den behandelten Sklerabereich des Auges (7) zu fokussieren: kegelförmig, kugelförmig, 90-Grad-Reflexionswinkel und flaches Ende.

8. Chirurgische Vorrichtung nach Anspruch 5, in der die Faserspitze (6) den Laserstrahl (1) auf den behandelten Bereich des Auges (7) mit einer Punktgröße von etwa 0,1 - 1,0) mm im Durchmesser fokussiert.

9. Chirurgische Vorrichtung nach Anspruch 5, in der die Faserspitze (6) in einer Zylinderform hergestellt ist, um den Laserstrahl (1) in einer Linienform, die eine Dimension von etwa (0,1 - 0,4) mm in der Breite und (0,5 - 4,0) mm in der Länge aufweist, auf den behandelten Bereich des Auges (7) zu fokussieren.

10. Chirurgische Vorrichtung nach Anspruch 5, in der die Faserspitze (6) in einem Kontaktmodus oder Nichtkontaktmodus operiert wird, um das Skleragewebe auf eine Tiefe von etwa (300 - 800) Mikrometer zu abladieren.

11. Chirurgische Vorrichtung nach Anspruch 1, in der die Faserzuführungseinheit (4,5,6) von dem Chirurgen steuerbar ist, um die vorbestimmten Muster (9) außerhalb des Limbus (8) der Kornea auszuführen, indem die Faserspitze (6) manuell in der Radialrichtung des Auges bewegt wird.

12. Chirurgische Vorrichtung nach Anspruch 1, in der die Faserzuführungseinheit (4,5,6) an eine Abtastvorrichtung befestigt ist, um die vorbestimmten Muster (9) außerhalb des Limbus (8) der Kornea auszuführen und den Laserstrahl (1) entlang der Radialrichtung des Auges zu bewegen.

13. Chirurgische Vorrichtung nach Anspruch 1, in der die vorbestimmten Muster außerhalb des Limbus (8) der Kornea sind und durch den Bereich zwischen zwei Kreisen mit einem Radius von etwa 5,0 mm bzw. 9,0 mm definiert sind.

14. Chirurgische Vorrichtung nach Anspruch 1, in der die vorbestimmten Muster (9) radiale Linien, kurvenförmige Linien, Ring-Punkt- oder nicht spezifische Muster um den Bereich außerhalb des Limbus (8) enthalten.

15. Chirurgische Vorrichtung nach Anspruch 1, in der der Ablationslaser einen ArF- oder XeCl-Excimerlaser oder einen Er:YAG-Laser oder einen Er:YSGG oder einen Nd:YAG-Laser oder einen Nd:YLF-Laser umfasst.

16. Chirurgische Vorrichtung nach Anspruch 1, in der das Steuermittel eine Abtastvorrichtung enthält, die einen motorisierten Reflexionsspiegel oder eine Brechungsoptik oder eine Translationsvorrichtung aufweist.

## Revendications

1. Appareil chirurgical pour traiter un patient presbytique en enlevant une partie du tissu scléral d'un oeil, comprenant:
(a) un laser pour émettre un faisceau laser (1) qui présente une longueur d'onde prédéterminée (2); et
(b) un mécanisme de contrôleur de spot de faisceau (3), ledit contrôleur de spot de faisceau étant adapté pour réduire et focaliser ledit faisceau laser (1) sur une unité de délivrance de fibre (4, 5, 6) ;
l'appareil chirurgical étant **caractérisé par**:
(c) un moyen de commande qui est adapté pour commander ladite unité de délivrance de fibre (4, 5, 6) afin de délivrer ledit faisceau laser (1) selon un motif prédéterminé (9) sur une pluralité de positions de l'oeil et afin de procéder à l'ablation du tissu scléral à l'extérieur de la zone de limbe jusqu'à une profondeur qui se situe entre environ 400 µm et environ 700 µm
et ainsi, l'appareil est adapté pour corriger une vision de patient presbytique en ce qui concerne la vue de près en augmentant l'accommodation de l'oeil par l'intermédiaire de l'augmentation de la flexibilité de la zone soumise à l'ablation qui est remplie par un tissu subconjonctival.

2. Appareil chirurgical selon la revendication 1, dans lequel ledit faisceau laser (1) est émis par un laser ultraviolet qui présente une plage de longueurs d'onde d'environ (0,15 - 0,36) µm et une durée d'impulsion qui est inférieure à environ 200 nanosecondes ou par un laser infrarouge qui présente une plage de longueurs d'onde d'environ (1,4 - 3,2) µm ou par un laser à diode à l'état solide qui présente une plage de longueurs d'onde d'environ (0,95 - 2,1) µm ou par un laser excimer ArF qui présente une longueur d'onde de 193 nanomètres ou par un laser excimer XeCl qui présente une longueur d'onde de 308 nanomètres.

3. Appareil chirurgical selon la revendication 2, dans lequel le laser infrarouge est un laser erbium:YAG pompé optiquement qui présente une longueur d'onde d'environ 2,9 µm ou un laser Er:YSGG pompé optiquement qui présente une longueur d'onde d'environ 2,7 µm.

4. Appareil chirurgical selon la revendication 1, dans lequel ledit contrôleur de spot de faisceau est constitué par au moins une lentille sphérique de focalisation (3) pour un couplage dudit faisceau laser (1) sur ladite unité de délivrance de fibre (4, 5, 6).

5. Appareil chirurgical selon la revendication 1, dans lequel ladite unité de délivrance de fibre (4, 5, 6) est constituée par une fibre optique (4) qui présente une longueur d'environ (0,5 - 1,5) mètre et un diamètre d'âme d'environ (0,2 - 0,8) millimètre et par une pièce à main qui est connectée à l'extrémité de fibre.

6. Appareil chirurgical selon la revendication 5, dans lequel ladite unité de délivrance de fibre (4, 5, 6) est sensiblement transparente à la longueur d'onde dudit faisceau laser (1).

7. Appareil chirurgical selon la revendication 5, dans lequel ladite extrémité de fibre (6) est réalisée en un matériau similaire à celui de la fibre (4) et est réalisée selon l'une des formes qui suivent de manière à focaliser ledit faisceau laser (1) sur la zone sclérale traitée de l'oeil (7): conique, sphérique, angle de réflexion de 90° et extrémité plane.

8. Appareil chirurgical selon la revendication 5, dans lequel ladite extrémité de fibre (6) focalise ledit faisceau laser (1) sur la zone traitée de l'oeil (7) selon une dimension de diamètre de spot d'environ (0,1 - 1,0) mm.

9. Appareil chirurgical selon la revendication 5, dans lequel ladite extrémité de fibre (6) est réalisée selon une forme cylindrique de manière à focaliser ledit faisceau laser (1) sur la zone traitée de l'oeil (7) selon une forme de ligne présentant une dimension de largeur d'environ (0,1 - 0,4) mm et une dimension de longueur d'environ (0,5 - 4,0) mm.

10. Appareil chirurgical selon la revendication 5, dans lequel ladite extrémité de fibre (6) est actionnée dans un mode contact ou dans un mode non contact de manière à réaliser l'ablation du tissu scléral jusqu'à une profondeur d'environ (300 - 800) µm.

11. Appareil chirurgical selon la revendication 1, dans lequel ladite unité de délivrance de fibre (4, 5, 6) peut être commandée par le chirurgien pour réaliser lesdits motifs prédéterminés (9) à l'extérieur du limbe (8) de la cornée en déplaçant manuellement ladite extrémité de fibre (6) suivant la direction radiale de l'oeil.

12. Appareil chirurgical selon la revendication 1, dans lequel ladite unité de délivrance de fibre (4, 5, 6) est fixée à un dispositif de balayage pour réaliser lesdits motifs prédéterminés (9) à l'extérieur du limbe (8) de la cornée et pour balayer ledit faisceau laser (1) suivant la direction radiale de l'oeil.

13. Appareil chirurgical selon la revendication 1, dans lequel lesdits motifs prédéterminés sont à l'extérieur du limbe (8) de la cornée et sont définis par la zone entre deux cercles présentant respectivement des rayons d'environ 5,0 mm et d'environ 9,0 mm.

14. Appareil chirurgical selon la revendication 1, dans lequel ledit motif prédéterminé (9) inclut des motifs en lignes radiales, en lignes courbes, en îlots en anneau ou des motifs non spécifiques autour de la zone à l'extérieur du limbe (8).

15. Appareil chirurgical selon la revendication 1, dans lequel ledit laser d'ablation comprend un laser excimer ArF ou XeCl ou un laser Er:YAG ou un laser Er:YSGG ou un laser Nd:YAG ou un laser Nd:YLF.

16. Appareil chirurgical selon la revendication 1, dans lequel ledit moyen de commande inclut un dispositif de balayage qui comporte un miroir de réflexion motorisé ou des optiques de réfraction motorisées ou un dispositif de translation motorisé.
